# EUROPEAN PATENT APPLICATION

(11) **EP 2 980 064 A1**
(43) Date of publication of application: **03.02.2016**
(21) Application number: 14775302.4
(22) Date of filing: 24.03.2014
(51) Int. Cl.: C07C 211/27, C07C 39/15, C08G 59/62

(54) **NOVEL INCLUSION COMPOUND**

(30) Priority: 25.03.2013 JP 2013061676
(71) Applicant: Nippon Soda Co., Ltd., Tokyo 100-8165 (JP)
(72) Inventor: ONO, Kazuo, Ichihara-shi Chiba 290-0045 (JP); NOMURA, Tomoya, Ichihara-shi Chiba 290-0045 (JP)
(74) Representative: Cabinet Plasseraud
(86) International application number: PCT/JP2014/001671
(87) International publication number: WO 2014/156103

(57) **Abstract**

An object of the present invention is to provide a novel curing agent or a curing accelerator for epoxy resin. The curing agent or a curing accelerator for epoxy resin of the present invention is a clathrate compound comprising the following (a1) and (a2):
(a1) a tetrakis(hydroxyphenyl)ethane compound or a carboxylic acid compound represented by formula:

A(COOH)ₖ

wherein, A represents a C1-C6 linear hydrocarbon group optionally having a substituent, a C3-C10 monocyclic hydrocarbon group optionally having a substituent, or a C6-C10 bicyclic hydrocarbon group optionally having a substituent, and k represents 2 or 3; and
(a2) a xylylene diamine compound.

## Description

### Technical Field

The present invention relates to a novel clathrate compound and a curing agent or a curing accelerator for epoxy resin comprising the compound.

The present application claims the priority of Japanese Patent Application No. 2013-061676 filed on March 25, 2013, the contents of which are incorporated herein by reference.

### Background Art

Epoxy resin has excellent mechanical properties and thermal properties and thus is widely used in various fields. The epoxy resin is utilized as adhesives for electronic components due to their intrinsic insulation properties and thermosetting properties, for example. Such thermosetting adhesives are required to stably exist in a state that thermosetting resin is not reacted with a curing agent until use, whereas the adhesives are required to cure in a short time in use. Thus, various curing agents and curing accelerators have been developed.

Patent document 1 discloses a curing agent for epoxy resin, consisting of a clathrate complex between a tetrakis phenolic compound and a compound that reacts with an epoxy group to thereby cure epoxy resin. However, epoxy resin had a high curing temperature and insufficiently cured at a low temperature.

Alternatively, patent document 2 discloses that a clathrate compound between 5-substituted isophthalic acid and imidazole is used as a curing agent or a curing accelerator for epoxy resin. However, the document provides no mention of the properties of clathrate compounds comprising an amine compound other than imidazole. Alternatively, patent document 3 discloses a latent curing agent that comprises a modified polyamine, phenol resin, and one or more polycarboxylic acids, but the curing agent was not a clathrate compound between the polyamine and the polycarboxylic acid and had insufficient storage stability.

### Prior Art Documents

### Patent Documents

Patent Document 1: Japanese unexamined Patent Application Publication No. 11-071449
Patent Document 2: Japanese unexamined Patent Application Publication No. 2007-039449
Patent Document 3: Japanese unexamined Patent Application Publication No. 2009-091460

### Summary of the Invention

### Object to be Solved by the Invention

It is an object of the present invention to provide a novel curing agent or a curing accelerator for epoxy resin.

### Means to Solve the Object

The present inventors have extensively studied to solve the problems described above and found that a curing agent or a curing accelerator for epoxy resin that is excellent in storage stability and curing properties can be provided by using a clathrate compound comprising a xylylene diamine compound and a tetrakis(hydroxyphenyl)ethane compound (referred to as a "TEP compound" hereinbelow) or a clathrate compound comprising a xylylene diamine compound and a carboxylic acid compound represented by A(COOH)ₖ, thereby having completed the present invention.

That is, the present invention relates to:
(1) A clathrate compound comprising the following (a1) and (a2):
   (a1) a tetrakis(hydroxyphenyl)ethane compound represented by formula (I): 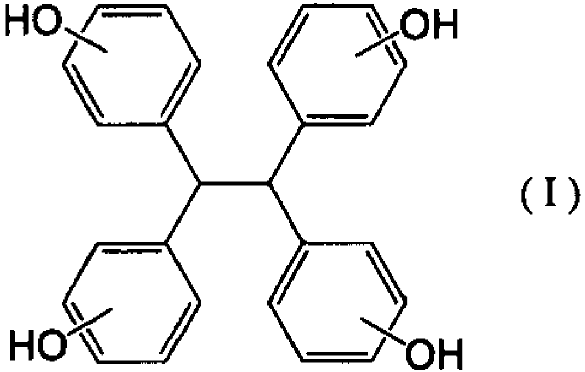 or a carboxylic acid compound represented by formula (II):

      A(COOH)ₖ (II)

      wherein A represents a C1-C6 linear hydrocarbon group optionally having a substituent, a C3-C10 monocyclic hydrocarbon group optionally having a substituent, or a C6-C10 bicyclic hydrocarbon group optionally having a substituent, and k represents 2 or 3; and
   (a2) a xylylene diamine compound represented by formula (III) :

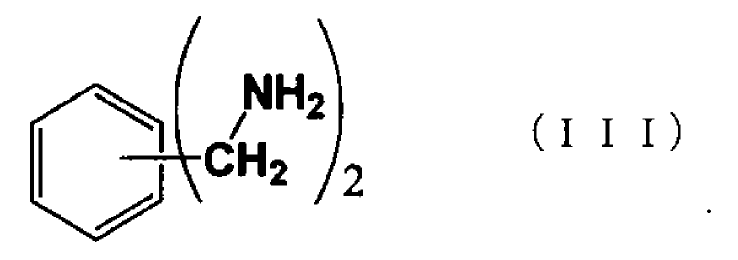

Also, the present invention relates to:
(2) the clathrate compound according to "1" described above, wherein (a1) is a tetrakis(hydroxyphenyl)ethane compound represented by formula (I): 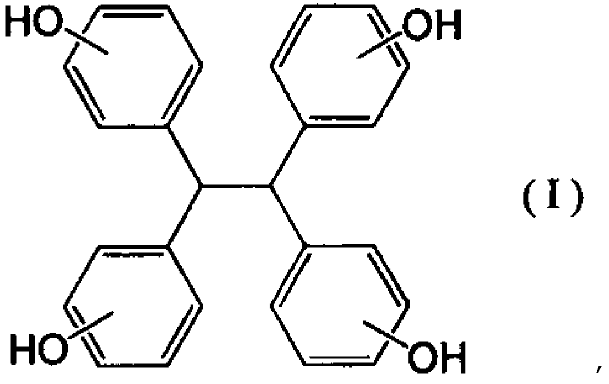
(3) the clathrate compound according to "1" described above, wherein (a1) is a carboxylic acid compound represented by formula (II):

   A(COOH)ₖ (II)

   wherein A represents a C1-C6 linear hydrocarbon group optionally having a substituent, a C3-C10 monocyclic hydrocarbon group optionally having a substituent, or a C6-C10 bicyclic hydrocarbon group optionally having a substituent, and k represents 2 or 3,
(4) the clathrate compound according to "3" described above, wherein the compound represented by formula (II) of (a1) is a compound represented by formula (IV) : 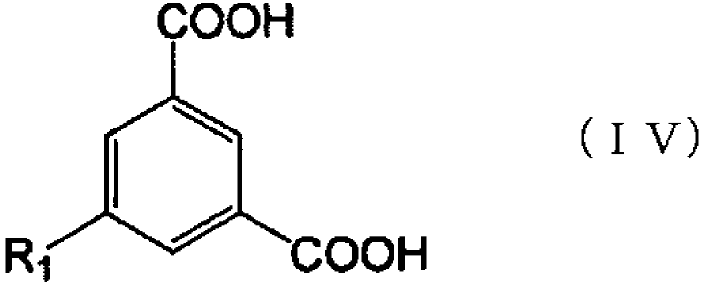 wherein R₁ represents a C1-C6 alkyl group, a C1-C6 alkoxy group, a nitro group, or a hydroxy group, and
(5) the clathrate compound according to any one of "1" to "4" described above, wherein the compound represented by formula (III) of (a2) is a compound represented by formula (V) :

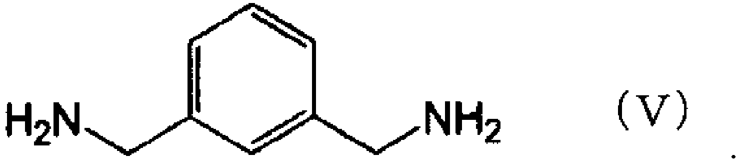

Furthermore, the present invention relates to: (6) a curing agent or a curing accelerator for epoxy resin comprising the clathrate compound according to any one of "1" to "5" described above.

### Effect of the Invention

A novel clathrate compound of the present invention can be used as a curing agent or a curing accelerator to provide a curing agent or a curing accelerator for epoxy resin that is excellent in storage stability and curing properties.

### Mode of Carrying Out the Invention

### (Clathrate compound)

The "clathrate compound" of the present invention refers to a compound formed by two or more molecules connected by weak bonding other than covalent bonding, more preferably to a crystalline compound formed by two or more molecules connected by weak bonding other than covalent bonding, and is a concept that includes salts for convenience. A compound to include is referred to as a host compound, and a compound to be included is referred to as a guest compound.

The clathrate compound of the present invention has a structure in which molecules of a host compound are connected to each other by weak bonding such as hydrogen bonding to form a structure and molecules of a guest compound are trapped into the spaces of the structure. The clathrate compound, at a certain temperature or higher, entirely releases the guest compound in a short time and serves as a curing agent/curing accelerator, allowing a good cured product to be obtained.

Particularly, when the clathrate compound of the present invention that comprises a TEP compound represented by formula (I) as the host compound and a xylylene diamine compound represented by formula (III) as the guest compound is used as a curing agent or a curing accelerator, not only the storage stability of the curing agent/curing accelerator is improved but also the epoxy resin can be cured at a lower temperature.

Alternatively, when a carboxylic acid compound represented by formula (II) is used as the host compound and a xylylene diamine compound represented by formula (III) is used as the guest compound, the clathrate compound will have a robust crystal structure due to the strong hydrogen bonding of the carboxylic acid. In the case where an epoxy resin composition is used as an adhesive, an organic solvent can be used for the purpose of reducing the viscosity. In this time, when a catalyst other than the product of the present invention (such as a microcapsule catalyst or a latent catalyst) is used, the catalyst dissolves in the organic solvent, and the epoxy resin reacts with the curing catalyst to cure. The product of the present invention is used, however, to provide a robust crystal structure, which has solvent resistance and allows storage for a long period as an epoxy resin composition in the presence of the organic solvent.

The clathrate compound of the present invention can comprise also a third component such as a solvent.

### (Guest compound)

The guest compound in the present invention is a xylylene diamine compound represented by the following formula (III):

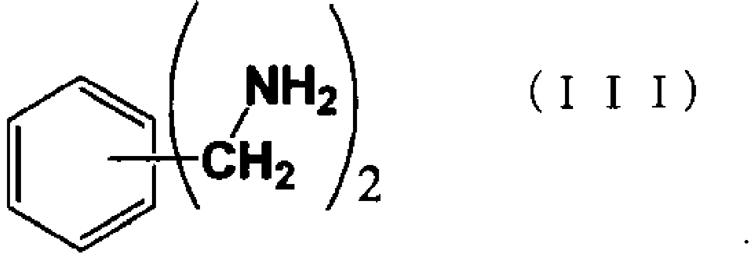

Specifically, examples of the xylylene diamine compound represented by formula (III) include o-xylylene diamine, m-xylylene diamine, and p-xylylene diamine. More preferably, the compound is a compound represented by formula (V):

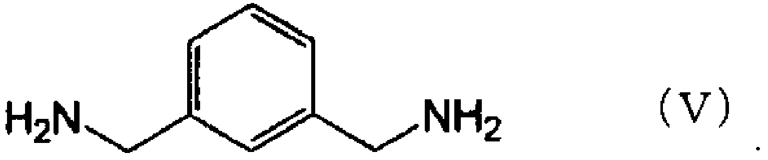

If the clathrate compound of the above-described xylylene diamine compound and a TEP compound or a carboxylic acid compound represented by A(COOH)ₖ falls within the above-described range, the combination is not particularly limited. These xylylene diamine compounds can be used singly, or two or more of the compounds can be used in combination.

### (Host compound)

The host compound of the present invention is a tetrakis(hydroxyphenyl)ethane compound represented by the following formula (I): 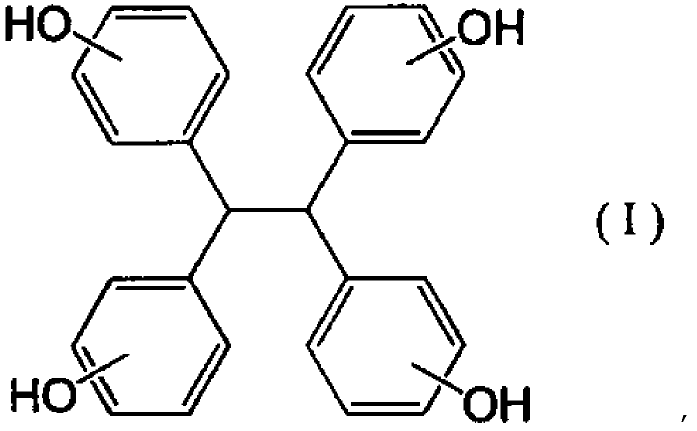 preferably 1,1,2,2-tetrakis(4-hydroxyphenyl)ethane.

Also, the host compound in the present invention is a carboxylic acid compound represented by formula (II):

A(COOH)ₖ (II).

A in A(COOH)ₖ represents a C1-C6 linear hydrocarbon group optionally having a substituent, a C3-C10 monocyclic hydrocarbon group optionally having a substituent, or a C6-C10 bicyclic hydrocarbon group optionally having a substituent, and k represents 2 or 3.

Examples of the C1-C6 linear hydrocarbon group of A include a divalent to trivalent group derived from a C1-C6 alkane, a C2-C6 alkene, or a C2-C6 alkyne.

Examples of the C1-C6 alkane include methane, ethane, n-propane, i-propane, n-butane, i-butane, n-pentane, and n-hexane.

Examples of the C2-C6 alkene include ethene, propene, 1-butene, 2-butene, 2-methyl-1-propene, 1-pentene, and 1-hexene.

Examples of the C2-C6 alkyne include ethyne, propyne, 1-butyne, 2-butyne, 1-pentyne, and 1-hexyne.

The C3-C10 monocyclic hydrocarbon group is a divalent to trivalent group derived from benzene or a C3-C10 alicyclic compound.

Examples of the C3-C10 alicyclic compound include a C3-C10 cycloalkane and a C3-C10 cycloalkene.

Examples of the C3-C10 cycloalkane include cyclopropane, cyclobutane, cyclopentane, cyclohexane, and cyclodecane.

Examples of the C3-C10 cycloalkene include cyclobutene, cyclopropene, cyclopentene, cyclohexene, and cyclooctene.

The C6-C10 bicyclic hydrocarbon group is a divalent to trivalent group derived from an aryl compound, or a fully-saturated or partially-unsaturated alicyclic compound.

Examples of the aryl compound include naphthalene, azulene, indene, indane, and tetralin.

Examples of the fully-saturated or partially-unsaturated alicyclic compound include bicyclo[2,2,0]hexane, bicyclo[2,2,1]heptane, bicyclo[4,1,0]heptan-2-ene, and bicyclo[3,2,0]heptan-2-ene.

Examples of the "substituent" in "optionally having a substituent" include a halogen atom, a C1-C6 alkyl group, an aryl group, a C1-C6 alkoxy group, a hydroxy group, a carboxyl group, a nitro group, an amino group, and an acyl group.

Examples of the C1-C6 alkyl group include a methyl group, an ethyl group, a n-propyl group, an i-propyl group, a n-butyl group, a s-butyl group, an i-butyl group, a t-butyl group, a n-pentyl group, a cyclobutyl group, a cyclopropyl methyl group, a n-pentyl group, an i-pentyl group, a 2-methylbutyl group, a neopentyl group, a 1-ethylpropyl group, a n-hexyl group, an i-hexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, a 1-ethylbutyl group, and a 2-ethylbutyl group.

Examples of the aryl group include a phenyl group, a benzyl group, a naphtyl group, an azulenyl group, an indenyl group, an indanyl group, and a tetralinyl group.

Examples of the C1-C6 alkoxy group include a methoxy group, an ethoxy group, a n-propoxy group, an i-propoxy group, a butoxy group, an i-butoxy group, a s-butoxy group, a t-butoxy group, a n-pentoxy group, an i-pentoxy group, a 2-methyl-n-butoxy group, a 1-ethyl-n-propoxy group, a 2-ethyl-n-propoxy group, a neopentoxy group, a n-hexyloxy group, a 4-methyl-n-pentoxy group, a 3-methyl-n-pentoxy group, a 2-methyl-n-pentoxy group, a 3,3-dimethyl-n-butoxy group, a 2,2-dimethyl-n-butoxy group, a 1,1-dimethyl-n-butoxy group, a 1,2-dimethyl-n-butoxy group, a 1,3-dimethyl-n-butoxy group, and a 2,3-dimethyl-n-butoxy group.

Examples of the above-described carboxylic acid compound represented by A(COOH)ₖ include an aromatic polyvalent carboxylic acid and an aliphatic polyvalent carboxylic acid.

The aromatic polyvalent carboxylic acid is preferably a compound represented by formula (IV): 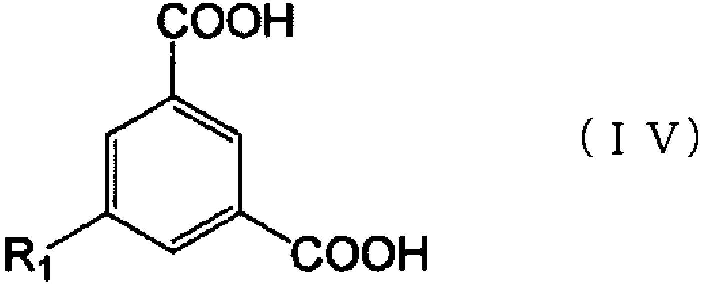 wherein R₁ represents a C1-C6 alkyl group, a C1-C6 alkoxy group, a nitro group, or a hydroxy group; and further, the compound represented by formula (IV) is more preferably 5-t-butyl isophthalic acid, 5-hydroxy isophthalic acid, or 5-nitro isophthalic acid.

Examples of the aliphatic polyvalent carboxylic acid include fumaric acid, 1,3-cyclohexanedicarboxylic acid, trans-1,4-cyclohexanedicarboxylic acid, succinic acid, malonic acid, tartaric acid, maleic acid, citric acid, malic acid, and adipic acid. The carboxylic acid is preferably a C2-C6 (carbon atoms in the carboxyl group are not included) aliphatic carboxylic acid having 2 or 3 carboxyl groups, or a hydroxy aliphatic polyvalent carboxylic acid.

These carboxylic acid compounds can be used singly, or two or more of the compounds can be used in combination.

### (Method for manufacturing a clathrate compound)

A method for manufacturing a clathrate compound can be obtained by directly mixing a xylylene diamine compound with a TEP compound or a carboxylic acid compound or by mixing the compounds in a solvent.

When a solvent is used, a clathrate compound can be obtained by adding a host compound and a guest compound to the solvent and then subjecting the resultant to heat treatment or heat reflux treatment under stirring as required followed by precipitation. Examples of the solvent that can be preferably used include, but are not particularly limited to, methanol, acetone, and ethyl acetate.

Formation of the clathrate compound can be confirmed by thermal analysis (TG and DTA), an infrared absorption spectrum (IR), an X ray diffraction pattern (XRD), a solid NMR spectrum, and the like. Also, the composition of the clathrate compound can be determined by thermal analysis, a ¹H-NMR spectrum, high performance liquid chromatography (HPLC), elementary analysis, and the like.

The ratio between the xylylene diamine compound and the TEP compound is not particularly limited as long as the compounds can form the clathrate compound, but the amount of the xylylene diamine compound is preferably 0.1 to 5.0 moles, more preferably 0.5 to 4.0 moles based on one mole of the TEP compound.

The ratio between the xylylene diamine compound and the carboxylic acid compound represented by A(COOH)ₖ is not particularly limited as long as the compounds can form the clathrate compound, but the amount of the xylylene diamine compound is preferably 0.1 to 5.0 moles, more preferably 0.5 to 4.0 moles based on one mole of the carboxylic acid compound.

The clathrate compound can additionally comprise a third component. In this case, the amount of the third component is preferably 40 mol% or less, more preferably 10 mol% or less, based on the total amount of the clathrate compound. In particular, it is most preferred that no third component be comprised.

The clathrate compound of the present invention can be used as a curing agent or a curing accelerator for epoxy resin.

As the epoxy resin, conventionally known various polyepoxy compounds can be used. Examples include an aromatic glycidyl ether compound, such as 2,2-bis(4-hydroxyphenyl)propane diglycidyl ether, 2,2-bis(hydroxyphenyl)propane diglycidyl ether, 2,2-bis(4-hydroxy-3,5-dibromophenyl)propane diglycidyl ether, 2,2-bis(4-hydroxyperhydrophenyl)propane diglycidyl ether, bis(4-hydroxyphenyl)sulfone diglycidyl ether, 2,2-bis(4-hydroxyphenyl)-1,1,1,3,3,3-hexafluoropropane diglycidyl ether, 3,5-dimethyl-4-hydroxybiphenyl diglycidyl ether, 1,2-bis(4-hydroxyphenyl)ethane diglycidyl ether, bis(4-hydroxyphenyl)methane diglycidyl ether, resorcinol diglycidyl ether, 1,6-dihydroxynaphthalene diglycidyl ether, 9,9-bis(4-hydroxyphenyl)fluorene diglycidyl ether, fluoroglycinol triglycidyl ether, trihydroxybiphenyl triglycidyl ether, 3,4,3',4'-tetraglycidyloxy benzophenone, bisresorcinol tetraglycidyl ether, tetramethyl bisphenol A diglycidyl ether, bisphenol C diglycidyl ether, 1,3-bis[1-(2,3-epoxypropoxy)-1-trifluoromethyl-2,2,2-trifluoroethyl]benzene, 1,4-bis[1-(2,3-epoxypropoxy)-1-trifluoromethyl-2,2,2-trifluoromethyl]benzene, 4,4'-bis(2,3-epoxypropoxy)octafluorobiphenyl, and a phenolnovolac-type bisepoxy compound,

an alicyclic polyepoxy compound, such as alicyclic diepoxy acetal represented by the following formula:

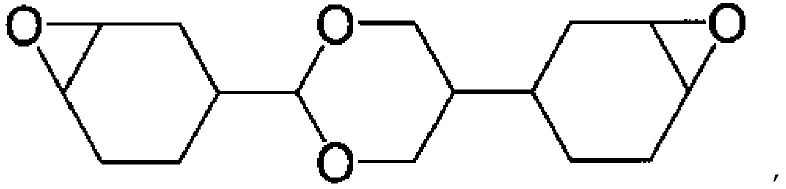

alicyclic diepoxy adipate represented by the following formula:

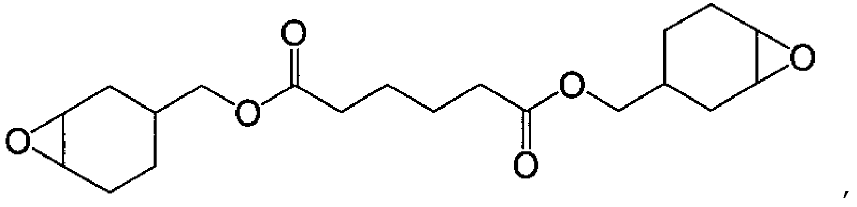

alicyclic diepoxy carboxylate represented by the following formula:

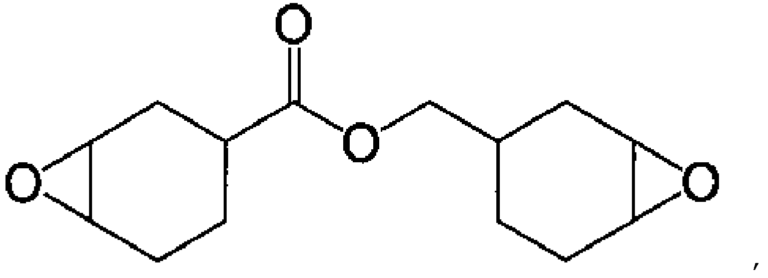

dicyclopentadiene dioxide represented by the following formula:

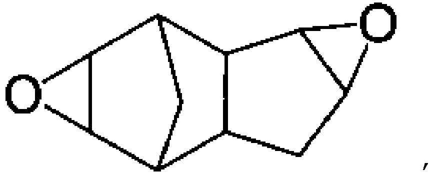

vinyl cyclohexene dioxide represented by the following formula:

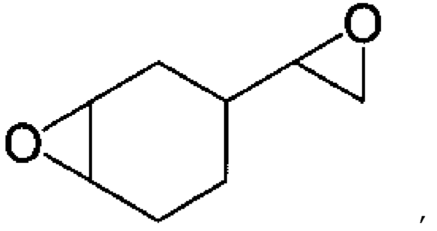

cyclohexene oxide glycidyl ether represented by the following formula: 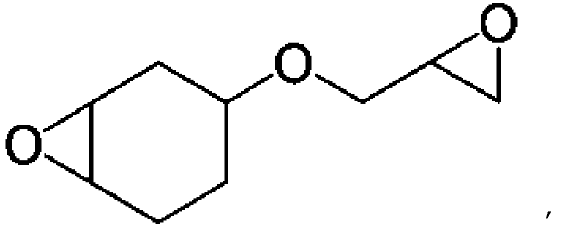 and

cyclohexene oxide glycidyl ester represented by the following formula:

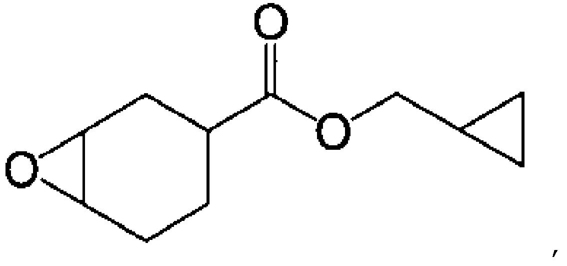

a glycidyl ester compound, such as diglycidyl phthalate, diglycidyl tetrahydrophthalate, diglycidyl hexahydrophthalate, dimethylglycidyl phthalate, dimethylglycidyl hexahydrophthalate, diglycidyl-p-oxybenzoate, diglycidyl cyclopentane-1,3-dicarboxylate, and a dimer acid glycidyl ester, a glycidyl amine compound, such as diglycidyl aniline, diglycidyl toluidine, triglycidyl aminophenol, tetraglycidyl diaminodiphenylmethane, and diglycidyl tribromoaniline, a heterocyclic epoxy compound, such as diglycidyl hydantoin, glycidyl glycidoxyalkyl hydantoin, and triglycidyl isocyanurate, and an oligomer compound thereof.

Examples of liquid epoxy resin include a polyalkylene ether-type epoxy compound, such as a (poly)ethylene glycol diglycidyl ether, a (poly)propylene glycol diglycidyl ether, and trimethylolpropane triglycidyl ether;
a glycidyl ester-type epoxy compound, such as a dimer acid diglycidyl ester, phthalic acid diglycidyl ester, and tetrahydrophthalic acid diglycidyl ester;
a polymer, such as glycidyl (meth)acrylate and an allylglycidyl ether or a copolymer of the monomer and other soft unsaturated monomer. The soft unsaturated monomer is a monomer of which homopolymer has a glass-transition temperature of less than 60°C, and examples include methyl acrylate, ethyl acrylate, butyl (meth)acrylate, isobutyl (meth)acrylate, 2-ethylhexyl (meth)acrylate, and lauryl methacrylate.

### (Applications of the epoxy resin composition)

The epoxy resin composition comprising the clathrate compound of the present invention can be used in various applications. The composition can be used in any applications in which epoxy resin can be generally used, for example, materials for electronic components such as encapsulants, adhesives, powder paint, and fiber reinforced plastics (FRP).

The epoxy resin comprising the clathrate compound of the present invention has improved storage stability compared to epoxy resin comprising only the guest compound. It is also possible to provide a curing agent or a curing accelerator having good curing properties such as a curing temperature and a curing rate.

### Examples

While Examples will be shown hereinbelow, the present invention is not bound by these examples in any way.

The clathrate compound can be also referred to as a catalyst or a curing catalyst hereinbelow.

Case of a clathrate compound of a xylylene diamine compound and a TEP compound

### (Host molecule)

TEP: 1,1,2,2-tetrakis(4-hydroxyphenyl)ethane

### (Guest molecule)

MXDA: m-xylylene diamine
2E4MZ: 2-ethyl-4-methyl imidazole
2P4MHZ: 2-phenyl-4-methyl-5-hydroxymethyl imidazole

Also, the notation of the clathrate compound is shown in the order from the host compound to the guest compound, and the clathrate ratio (molar ratio) between the host compound and the guest compound is shown in the subsequent parentheses. For example, "TEP-MXDA (1/1)" means a clathrate compound in which the host compound is TEP and the guest compound is MXDA, and the clathrate ratio is 1/1.

### [Example 1] TEP-MXDA (1/2)

To 7.97 g of TEP, 50 mL of ethyl acetate was added, and the resulting mixture was stirred. A solution of 5.45 g of MXDA diluted with 10 mL of ethyl acetate was added dropwise thereto, and after the addition, the resulting mixture was heated to reflux for three hours. After cooling, filtration and vacuum drying were carried out to thereby yield a clathrate compound, TEP-MXDA (1/2). Yield: 13.13 g. The clathrate compound obtained was subjected to ¹H-NMR, TG-DTA, and XRD to confirm clathrate. Additionally, the decomposition point was 178-183°C.

### (Curing start temperature of liquid curable epoxy resin)

### [Measurement Example 1]

To 5 g of an epoxy resin, EPOTOHTO YD-128 (R: manufactured by NIPPON STEEL & SUMIKIN CHEMICAL CO., LTD., epoxy equivalent 184-194 g/eq), 0.2 g of MXDA was mixed to thereby yield a curable epoxy resin composition. The composition was measured by DSC (manufactured by TA instruments, model Q2000) for the curing start temperature of the epoxy resin (extrapolated peak start temperature). The result is shown in Table 1.

### [Measurement Example 2]

To 5 g of an epoxy resin, EPOTOHTO YD-128 (R: manufactured by NIPPON STEEL & SUMIKIN CHEMICAL CO., LTD., epoxy equivalent 184-194 g/eq), 0.49 g of TEP-MXDA (1/2) (Example 1) was mixed to thereby yield a curable epoxy resin composition. The composition was measured by DSC (manufactured by TA instruments, model Q2000) for the curing start temperature of the epoxy resin (extrapolated peak start temperature). The result is shown in Table 1.

### [Measurement Example 3]

To 5 g of an epoxy resin, EPOTOHTO YD-128 (R: manufactured by NIPPON STEEL & SUMIKIN CHEMICAL CO., LTD., epoxy equivalent 184-194 g/eq), 0.56 g of TEP-2E4MZ (1/2) was mixed to thereby yield a curable epoxy resin composition. The composition was measured by DSC (manufactured by TA instruments, model Q2000) for the curing start temperature of the epoxy resin (extrapolated peak start temperature). The result is shown in Table 1.

### [Measurement Example 4]

To 5 g of an epoxy resin, EPOTOHTO YD-128 (R: manufactured by NIPPON STEEL & SUMIKIN CHEMICAL CO., LTD., epoxy equivalent 184-194 g/eq), 0.41 g of TEP-2P4MHZ (1/2) was mixed to thereby yield a curable epoxy resin composition. The composition was measured by DSC (manufactured by TA instruments, model Q2000) for the curing start temperature of the epoxy resin (extrapolated peak start temperature). The result is shown in Table 1.

**[Table 1] Curing start temperature**

| | Compound | Curing start temperature |
|---|---|---|
| Measurement example 1 | MXDA | 76°C |
| Measurement example 2 | TEP-MXDA (1/2) | 102°C |
| Measurement example 3 | TEP-2E4MZ (1/2) | 137°C |
| Measurement example 4 | TEP-2P4MHZ (1/2) | 158°C |

### (Storage stability of liquid curable epoxy resin)

### [Measurement Example 5]

To 10 g of an epoxy resin, EPOTOHTO YD-128 (R: manufactured by NIPPON STEEL & SUMIKIN CHEMICAL CO., LTD., epoxy equivalent 184-194 g/eq), 0.4 g of MXDA was mixed to thereby yield a curable epoxy resin composition. The composition was stored at 30°C and visually observed. Measurement was ended when the composition solidified, and the storage stability was evaluated. The result is shown in Table 2.

### [Measurement Example 6]

To 10 g of an epoxy resin, EPOTOHTO YD-128 (R: manufactured by NIPPON STEEL & SUMIKIN CHEMICAL CO., LTD., epoxy equivalent 184-194 g/eq), 0.99 g of TEP-MXDA (1/2) was mixed to thereby yield a curable epoxy resin composition. The composition was stored at 30°C and visually observed. Measurement was ended when the composition solidified, and the storage stability was evaluated. The result is shown in Table 2.

**[Table 2] Storage stability**

| Example | Compound | Storage stability |
|---|---|---|
| Measurement example 5 | MXDA | Solidifies in two days |
| Measurement example 6 | TEP-MXDA (1/2) | Solidifies in 10 days |

In comparison of Measurement Example 1 with Measurement Example 2, the composition in which only the guest compound was used as the curing agent can be cured at a lower temperature. However, in comparison of Measurement Example 5 with Measurement Example 6, the epoxy resin composition in which only the guest compound was used as the curing agent started curing reaction at 30°C and solidified in two days, causing an industrial problem. If the temperature rises at a rate of 10°C/minute, the curing reaction starts at 76°C. Indeed, the curing reaction occurs even at 30°C, and thus, the guest compound, when used as the curing agent, has to be used immediately after mixed with the epoxy resin. However, the clathrate compound of the present invention has sufficient time until it solidifies, causing no industrial problem.

Alternatively, in comparison with the known clathrate compounds (Measurement Example 3 and Measurement Example 4), it was found that the epoxy resin composition in which the clathrate compound of the present invention (Measurement Example 2) was used as the curing agent had a lower curing start temperature, despite of use of the same TEP as the host compound. Use of the clathrate compound of the present invention as the curing agent enables the epoxy resin to cure at a lower temperature to thereby minimize thermal damage on the epoxy resin. This makes the compound suitable for use for thermally-susceptible epoxy resin. Also, a low heat-resistant material can be used as an adherend for epoxy resin.

Case of a clathrate compound of a xylylene diamine compound and a carboxylic acid compound

### (Host molecule)

NIPA: 5-nitroisophthalic acid
HIPA: 5-hydroxyisophthalic acid

### (Guest molecule)

MXDA: m-xylylene diamine

Also, the notation of the clathrate compound is shown in the order from the host compound to the guest compound, and the clathrate ratio (molar ratio) between the host compound and the guest compound is shown in the subsequent parentheses. For example, "HIPA-MXDA (1/1)" means a clathrate compound in which the host compound is HIPA and the guest compound is MXDA, and the clathrate ratio is 1/1.

### [Example 2]

### (HIPA-MXDA)

To 7.29 g of HIPA, 40 mL of methanol was added, and the resulting mixture was stirred for dissolution. A solution of 5.45 g of MXDA diluted with 10 mL of methanol was added dropwise thereto, and the resulting mixture was heated to reflux, on completion of the dropwise addition, for three hours. After cooling, filtration and vacuum drying were carried out to thereby yield a clathrate compound, HIPA-MXDA (1/1). The clathrate compound obtained was subjected to ¹H-NMR, TG-DTA, and XRD to confirm clathrate. Decomposition point: 235-240°C.

### [Example 3]

### (NIPA-MXDA)

To 8.45 g of NIPA, 40 mL of ethyl acetate was added, and the resulting mixture was stirred for dissolution. A solution of 5.45 g of MXDA diluted with 10 mL of ethyl acetate was added dropwise thereto, and the resulting mixture was heated to reflux, on completion of the dropwise addition, for three hours. After cooling, filtration and vacuum drying were carried out to thereby yield a clathrate compound, NIPA-MXDA (1/1). The clathrate compound obtained was subjected to ¹H-NMR, TG-DTA, and XRD to confirm clathration. Decomposition point: 265-270°C.

### (Curing start temperature of liquid curable epoxy resin)

### [Measurement Example 7]

To 5 g of an epoxy resin, EPOTOHTO YD-128 (R: manufactured by NIPPON STEEL & SUMIKIN CHEMICAL CO., LTD., epoxy equivalent 184-194 g/eq), 0.2 g of MXDA was mixed to thereby yield a curable epoxy resin composition. The composition was measured by DSC (manufactured by TA instruments, model Q2000) for the curing start temperature of the epoxy resin (extrapolated peak start temperature). The result is shown in Table 3.

### [Measurement Example 8]

To 5 g of an epoxy resin, EPOTOHTO YD-128 (R: manufactured by NIPPON STEEL & SUMIKIN CHEMICAL CO., LTD., epoxy equivalent 184-194 g/eq), 0.47 g of HIPA-MXDA (Example 1) was mixed to thereby yield a curable epoxy resin composition. The composition was measured by DSC (manufactured by TA instruments, model Q2000) for the curing start temperature of the epoxy resin (extrapolated peak start temperature). The result is shown in Table 3.

### [Measurement Example 9]

To 5 g of an epoxy resin, EPOTOHTO YD-128 (R: manufactured by NIPPON STEEL & SUMIKIN CHEMICAL CO., LTD., epoxy equivalent 184-194 g/eq), 0.51 g of NIPA-MXDA (Example 2) was mixed to thereby yield a curable epoxy resin composition. The composition was measured by DSC (manufactured by TA instruments, model Q2000) for the curing start temperature of the epoxy resin (extrapolated peak start temperature). The result is shown in Table 3.

**[Table 3] Curing start temperature**

| | Compound | Curing start temperature |
|---|---|---|
| Measurement example 7 | MXDA | 76°C |
| Measurement example 8 | HIPA-MXDA (1/1) | 196°C |
| Measurement example 9 | NIPA-MXDA (1/1) | 189°C |

In comparison with the guest compound which is not included (Measurement Example 7), the epoxy resin compositions in which the clathrate compound of the present invention (Measurement Example s 8 and 9) is used have a higher curing start temperature. This has revealed that the epoxy resin compositions in which the clathrate compound of the present invention is used have improved heat resistance.

### (Storage stability of liquid curable epoxy resin)

### [Measurement Example 10]

To 10 g of an epoxy resin EPOTOHTO YD-128 (R: manufactured by NIPPON STEEL & SUMIKIN CHEMICAL CO., LTD., epoxy equivalent 184-194 g/eq), 0.4 g of MXDA was mixed to thereby yield a curable epoxy resin composition. The composition was stored at 30°C and visually observed. Measurement was ended when the composition solidified, and the storage stability was evaluated. The result is shown in Table 4.

### [Measurement Example 11]

To 10 g of an epoxy resin, EPOTOHTO YD-128 (R: manufactured by NIPPON STEEL & SUMIKIN CHEMICAL CO., LTD., epoxy equivalent 184-194 g/eq), 0.93 g of HIPA-MXDA was mixed to thereby yield a curable epoxy resin composition. The composition was stored at 30°C and visually observed. Measurement was ended when the composition solidified, and the storage stability was evaluated. The result is shown in Table 4.

### [Measurement Example 12]

To 10 g of an epoxy resin, EPOTOHTO YD-128 (R: manufactured by NIPPON STEEL & SUMIKIN CHEMICAL CO., LTD., epoxy equivalent 184-194 g/eq), 1.02 g of NIPA-MXDA was mixed to thereby yield a curable epoxy resin composition. The composition was stored at 30°C and visually observed. Measurement was ended when the composition solidified, and the storage stability was evaluated. The result is shown in Table 4.

**[Table 4] Storage stability**

| | Compound | Storage stability |
|---|---|---|
| Measurement example 10 | MXDA | Solidifies in two days |
| Measurement example 11 | HIPA-MXDA (1/1) | Not solidifies in 100 days |
| Measurement example 12 | NIPA-MXDA (1/1) | Not solidifies in 100 days |

In comparison with MXDA which is not included, it has been revealed that the epoxy resin composition in which the clathrate MXDA is used has significantly improved storage stability.

### Industrial Applicability

The novel clathrate compound of the present invention can be used as a curing agent, a curing accelerator, and the like for epoxy resin.

## Claims

1. A clathrate compound comprising the following (a1) and (a2) :
(a1) a tetrakis(hydroxyphenyl)ethane compound represented by formula (I): 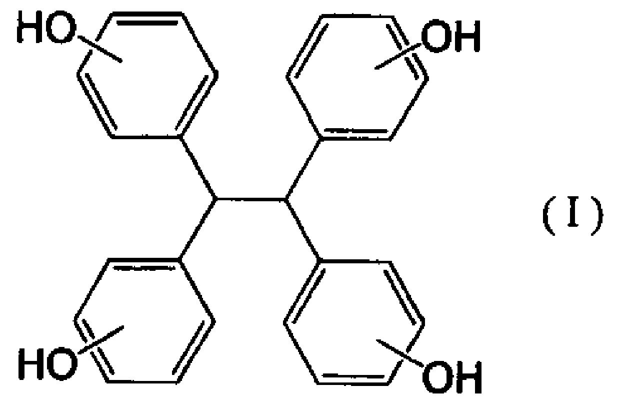 or a carboxylic acid compound represented by formula (II):
A(COOH)ₖ (II)
wherein A represents a C1-C6 linear hydrocarbon group optionally having a substituent, a C3-C10 monocyclic hydrocarbon group optionally having a substituent, or a C6-C10 bicyclic hydrocarbon group optionally having a substituent, and k represents 2 or 3; and
(a2) a xylylene diamine compound represented by formula (III) :

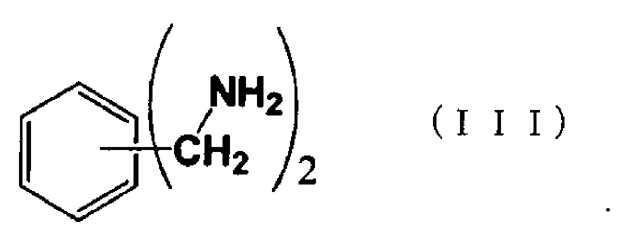

2. The clathrate compound according to claim 1, wherein (a1) is a tetrakis(hydroxyphenyl)ethane compound represented by formula (I):

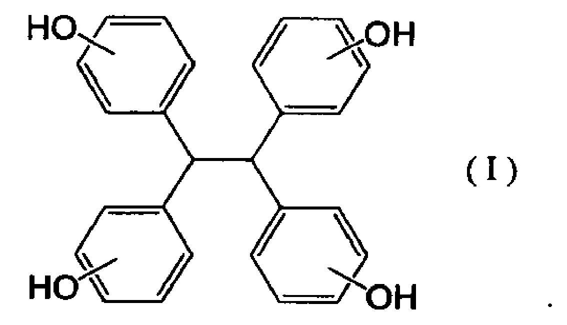

3. The clathrate compound according to claim 1, wherein (a1) is a carboxylic acid compound represented by formula (II) :
A(COOH)ₖ (II)
wherein A represents a C1-C6 linear hydrocarbon group optionally having a substituent, a C3-C10 monocyclic hydrocarbon group optionally having a substituent, or a C6-C10 bicyclic hydrocarbon group optionally having a substituent, and k represents 2 or 3.

4. The clathrate compound according to claim 3, wherein the compound represented by formula (II) of (a1) is a compound represented by formula (IV): 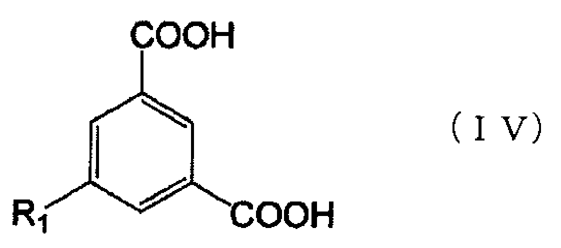 wherein R₁ represents a C1-C6 alkyl group, a C1-C6 alkoxy group, a nitro group, or a hydroxy group.

5. The clathrate compound according to any one of claims 1 to 4, wherein the compound represented by formula (III) of (a2) is a compound represented by formula (V):

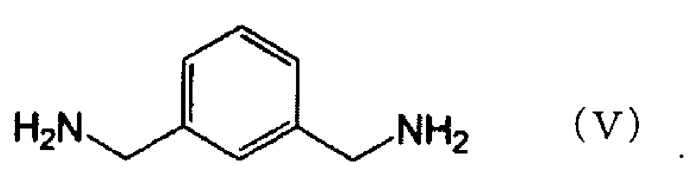

6. A curing agent or a curing accelerator for epoxy resin comprising the clathrate compound according to any one of claims 1 to 5.
